(19) Europäisches Patentamt
European Patent Office
Office européen des brevets

(11) Veröffentlichungsnummer: **0 078 450**
**B1**

(12) # EUROPÄISCHE PATENTSCHRIFT

(45) Veröffentlichungstag der Patentschrift: **02.10.85**

(51) Int. Cl.⁴: **C 07 D 237/18**, A 01 N 43/58

(21) Anmeldenummer: **82109671.6**

(22) Anmeldetag: **20.10.82**

(54) **Pyridazinon-Derivate, Verfahren zu ihrer Herstellung und ihre Verwendung als Fungizide.**

(30) Priorität: **31.10.81 DE 3143303**

(43) Veröffentlichungstag der Anmeldung: **11.05.83 Patentblatt 83/19**

(45) Bekanntmachung des Hinweises auf die Patenterteilung: **02.10.85 Patentblatt 85/40**

(84) Benannte Vertragsstaaten: **AT BE CH DE FR GB IT LI NL SE**

(56) Entgegenhaltungen:
**DE - A - 1 445 475**

**CHEMICAL ABSTRACTS, Band 72, 1970, Seite 424, Nr. 55374s, Columbus, Ohio, USA T. TAKAHASHI et al.: "Pyridazinone derivatives. IV. Synthesis, antibacterial, and antifungal activity of sulfur-containing pyridazinone derivatives"**
**CHEMICAL ABSTRACTS, Band 70, 1969, Seite 394, Nr. 68398m, Columbus, Ohio, USA**
**CHEMICAL ABSTRACTS, Band 75, 1971, Seite 230, Nr. 129060x, Columbus, Ohio, USA V. MAKI et al.: "Ring contraction of pyridazinones to pyrazoles. V."**
**CHEMICAL ABSTRACTS, Band 87, 1977, Seite 734, Nr. 201567k, Columbus, Ohio, USA**
**CHEMICAL ABSTRACTS, Band 88, 1978, Seite 507, Nr. 37821m, Columbus, Ohio, USA**

(73) Patentinhaber: **BASF Aktiengesellschaft, Carl-Bosch-Strasse 38, D-6700 Ludwigshafen (DE)**

(72) Erfinder: **Richarz, Winfried, Dr., Königsberger Strasse 5, D-6081 Stockstadt am Rhein (DE)**
Erfinder: **Reissenweber, Gernot, Dr., Drosselstrasse 15, D-6737 Böhl-Iggelheim (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

(56) Entgegenhaltungen: (Fortsetzung)
**CHEMICAL ABSTRACTS, Band 70, 1969, Seite 394, Nr. 68397k, Columbus, Ohio, USA**
**CHEMICAL ABSTRACTS, Band 90, 1979, Seite 618, Nr. 87377h, Columbus, Ohio, USA M. TAKAYA et al.: "Studies on pyridazinone derivatives. IX. Synthesis and hemostatic activity of 2,4,5-trisubstituted-3(2H9-pyridazinones"**

**EP 0 078 450 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue in 4-Stellung durch Sulfinyl- bzw. Sulfonyl-Gruppen substituierte Pyridazinon-Derivate, Verfahren zu ihrer Herstellung, Fungizide, die diese Verbindungen als Wirkstoff enthalten, sowie Verfahren zur Bekämpfung von Pilzen mit ihnen.

Aus der DE-OS 1 445 475 ist es bekannt, daß Pyridazinon-Derivate der Formel

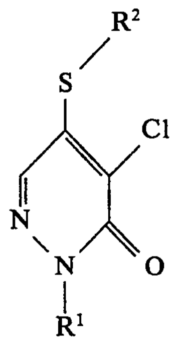

in der $R^1$ einen gegebenenfalls substituierten Alkyl-, Cycloalkyl- oder Arylrest und $R^2$ Wasserstoff oder einen gegebenenfalls substituierten Alkyl-, Alkenyl-, Cycloalkyl-, Acyl- oder Aralkylrest bedeuten, als Mittel zur Beeinflussung des Pflanzenwachstums geeignet sind.

Weiterhin ist aus Yakugaku Zasshi 89, 1516—1527 (1969) bekannt, daß 1-Phenyl-4-methylsulfonyl-5-chlor-pyridazinon-(6) eine bakterizide und fungizide Wirkung besitzt.

Es sind ferner 4-Methylsulfonyl-5-chlor-pyridazone bekannt, die in 1-Stellung den Phenyl-, 4-Nitrophenyl- oder 2,4-Dinitrophenylrest enthalten und pharmazeutisch wirksam sind (C.A. 70, 394 Nr. 68398 m (1969), C.A. 70, 394 Nr. 68397 k (1969), C.A. 87, 734 Nr. 201567 k (1977), C.A. 88, 507 Nr. 37821 m (1978)).

Es wurde gefunden, daß Pyridazinon-Derivate der allgemeinen Formel I

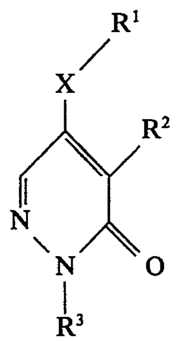

(I)

in der

$R^1$ Methyl, Ethyl, Propyl, i-Propyl, tert.-Butyl, Cyclohexyl, Phenyl oder p-Chlorphenyl und
$R^2$ Chlor oder Brom und
$R^3$ Wasserstoff, Methyl, Ethyl, i-Propyl, Cyclohexyl, Phenyl, 4-Chlorphenyl, 3,5-Dichlorphenyl, 4-Bromphenyl, 3-Trifluormethylphenyl, 3-Methylphenyl, 4-Methylphenyl, 4-Acetoxiphenyl oder 3-(Pentafluorethoxi)-phenyl und
X SO oder $SO_2$ bedeuten mit der Maßgabe, daß $R^1$ nicht Methyl bedeutet, wenn X $SO_2$, $R^2$ Chlor und $R^3$ Phenyl bedeuten, fungizid ausgezeichnet wirksam sind.

Die Pyridazinon-Derivate der Formel I können durch Oxidation von Pyridazinonen der Formel II

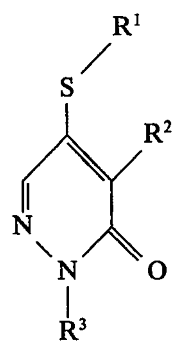

(II)

in der $R^1$, $R^2$ und $R^3$ die obengenannten Bedeutungen haben, mit Wasserstoffperoxid oder Persäuren, ggf. in Gegenwart eines Lösungs- oder Verdünnungsmittels, bei einer Temperatur im Bereich von 0 bis 100°C erhalten werden. Als Lösungs- oder Verdünnungsmittel finden beispielsweise Eisessig oder Aceton Verwendung. Zweckmäßig verwendet man beispielsweise zur Herstellung der Pyridazinone der Formel I mit X = SO etwa 1,0 bis 1,1 Mol Oxidationsmittel (Wasserstoffperoxid oder Persäure) pro Mol eines Pyridazinons der Formel II und zur Herstellung der Pyridazinone der Formel I mit X = $SO_2$ 2,0 bis 5,0 Mol Oxidationsmittel pro Mol eines Pyridazinons der Formel II. Die Umsetzung wird im allgemeinen bei einer Temperatur im Bereich von 0 bis 100°C, beispielsweise in einem Zeitraum von 1 bis 100 Stunden, drucklos oder unter Druck, kontinuierlich oder diskontinuierlich durchgeführt.

In einer bevorzugten Ausführungsform des erfindungsgemäßen Verfahrens gibt man zur Lösung des Ausgangsstoffs der Formel II in Eisessig Wasserstoffperoxid zu und hält das Reaktionsgemisch für 1 bis 100 Stunden, vorzugsweise 4 bis 20 Stunden, bei einer Reaktionstemperatur, die zwischen 0 und 100°C, vorzugsweise 60 bis 80°C, liegen kann.

Zur Isolierung der Pyridazinone der Formel I kühlt man beispielsweise das Reaktionsgemisch auf 0°C ab und saugt die sich abscheidenden Kristalle ab. Die Mutterlauge kann für eine weitere Umsetzung wieder verwendet werden. Eine weitere Möglichkeit zur Isolierung der neuen Substanzen besteht darin, das Reaktionsgemisch mit Wasser zu verdünnen und den dabei ausfallenden Niederschlag abzusaugen. Die nach Waschen mit Wasser verbleibenden Produkte bedürfen im allgemeinen keiner weiteren Reinigung, können aber, falls erforderlich, nach bekannten Methoden, beispielsweise durch Umkristallisation, Extraktion oder Chromatographie, weiter gereinigt werden.

Die zur Herstellung der Pyridazinone der Formel I dienenden Pyridazinone der Formel II können durch Umsetzung eines Pyridazinons der Formel III

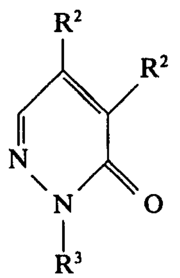

(III)

in der $R^2$ und $R^3$ die obengenannten Bedeutungen haben, mit einem Thiol der Formel III

$R^1-SH$ (IV)

in der $R^1$ die obengenannte Bedeutung hat, ggf. in Gegenwart eines Lösungs- oder Verdünnungsmittels, in Anwesenheit anorganischer oder organischer Basen und ggf. in Anwesenheit eines Reaktionsbeschleunigers, bei einer Temperatur im Bereich zwischen −20°C und +120°C erhalten werden.

Die Umsetzung kann in Gegenwart eines Lösungs- oder Verdünnungsmittels durchgeführt werden. Zu den bevorzugten Lösungs- bzw. Verdünnungsmitteln gehören Halogenkohlenwasserstoffe, beispielsweise Methylenchlorid, Chloroform, 1,2-Dichlorethan, Chlorbenzol; aliphatische oder aromatische Kohlenwasserstoffe, wie Cyclohexan, Petrolether, Benzol, Toluol oder Xylole; Ester, wie Essigsäureethylester; Nitrile, wie Acetonitril; Sulfoxide, wie Dimethylsulfoxid; Ketone, wie Aceton oder Methylethylketon; Ether, wie Diethylether, Tetrahydrofuran oder Dioxan oder Gemische dieser Lösungsmittel.

Zweckmäßig verwendet man das Lösungs- bzw. Verdünnungsmittel in einer Menge von 100 bis 2000 Gew.-%, vorzugsweise 100 bis 1000 Gew.-%, bezogen auf die Ausgangsstoffe der Formel II bzw. III.

Geeignete anorganische oder organische Basen, die ggf. als säurebindende Mittel dem Reaktionsgemisch zugesetzt werden können, sind beispielsweise Alkalicarbonate, wie Kalium- oder Natriumcarbonat; Alkalihydride, wie Natriumhydrid; tertiäre Amine, wie Trimethylamin, Triethylamin, N,N-Dimethyl anilin, N,N-Dimethylcyclohexylamin, N-Methylpiperidin oder Pyridin; Azole, wie 1,2,4-Triazol oder Imidazol. Es können aber auch andere übliche Basen verwendet werden. Als Reaktionsbeschleuniger kommen vorzugsweise Metallhalogenide, wie Natriumbromid oder Kaliumjodid, Azole, wie Imidazol oder Pyridine wie 4-Methyl-pyridin oder quartäre Ammoniumsalze wie N,N-Dibenzyl-N,N-dimetylammoniumchlorid oder Mischungen dieser Substanzen in Betracht.

Zweckmäßig setzt man auf 1 Mol Pyridazinon der Formel III 1,0 bis 2,0 Mol Thiol der Formel IV sowie ggf. 0,5 bis 2 Mol Base und ggf. 0,01 Mol bis 0,1 Mol Reaktionsbeschleuniger ein.

Die Umsetzung wird im allgemeinen bei einer Temperatur im Bereich von −20 und +120°C, beispielsweise in einem Zeitraum von 1 bis 80 Stunden durchgeführt, drucklos oder unter Druck, kontinuierlich oder diskontinuierlich.

In einer bevorzugten Form des erfindungsgemäßen Verfahrens vermischt man die Lösung des Ausgangsstoffs der Formel III in einem der angegebenen Lösungs- oder Verdünnungsmittel mit verdünntem wäßrigen Alkali und ggf. dem Reaktionsbeschleuniger in Form eines quartären Ammonium-Salzes, gibt das Thiol-Derivat zu und hält das Reaktionsgemisch für 0,5 bis 12, vorzugsweise 1 bis 6 Stunden bei der Reaktionstemperatur, die zwischen −20°C und +120°C liegen kann.

Zur Isolierung der Thiol-pyridazinone der Formel II trennt man die organische Phase ab und wäscht diese mit Wasser. Die nach dem Abdestillieren des Lösungsmittels verbleibenden Produkte können, falls erforderlich, nach bekannten Methoden durch Umkristallisation, Extraktion oder Chromatographie weiter gereinigt werden.

Die als Ausgangsstoffe eingesetzten Pyridazinon-Derivate der allgemeinen Formel III sind teilweise bekannt bzw. können nach bekannten Verfahren durch Umsetzung von Mucochlor- bzw. Mucobromsäure mit einem Hydrazin der Formel V

$$H_2N-NH-R^3 \qquad (V)$$

in der $R^3$ die oben angegebene Bedeutung hat, hergestellt werden.

Die folgende Vorschrift erläutert die Herstellung der zur Gewinnung der neuen Wirkstoffe der Formel I notwendigen Pyridazinon-Derivate der Formel II. Gewichtsteile verhalten sich zu Volumenteilen wie kg zu Liter.

Vorschrift

In eine Mischung aus 72,3 Gew.-Teilen 1-Phenyl-4,5-dichlorpyridazinon-6 in 300 Vol.-Teilen Methylenchlorid, 3 Gew.-Teilen Triethylbenzylammoniumchlorid und 13,2 Gew.-Teilen Natriumhydroxid in 150 Vol.-Teilen Wasser tropft man bei Raumtemperatur (20°C) 38,3 Gew.-Teile Cyclohexylthiol und hält die Reaktionsmischung unter kräftigem Rühren 12 h bei Raumtemperatur. Die organische Phase wird abgetrennt und zweimal mit je 100 Vol.-Teilen Wasser gewaschen, anschließend getrocknet und eingeengt. Der verbleibende Rückstand wird aus Essigester umkristallisiert. Man erhält 61 Gew.-Teile 1-Phenyl-4-(cyclohexylthio)-5-chlorpyridazinon-6 vom Schmp. 125 bis 127°C (Verbindung AI).

$C_{16}H_{17}ClN_2SO$ (320,5):

| | | | | |
|---|---|---|---|---|
| Ber.: | C 59,9 | H 5,34 | N 8,73 | S 9,99 |
| Gef.: | C 60,0 | H 5,5 | N 8,7 | S 10,1 |

Entsprechend lassen sich folgende Verbindungen der Formel II herstellen.

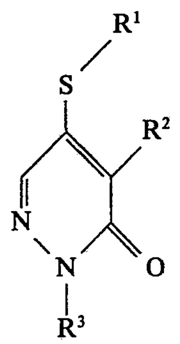

(II)

| Nr. | $R^1$ | $R^2$ | $R^3$ | Fp. (°C) |
|---|---|---|---|---|
| A 2 | $CH_3$ | Cl | $C_6H_5$ | 116–117 |
| A 3 | $C_2H_5$ | Cl | $C_6H_5$ | 92– 93 |
| A 4 | $C_2H_5$ | Cl | 4-Chlorphenyl | 140–142 |
| A 5 | $C_2H_5$ | Cl | 4-Bromphenyl | 135–136 |
| A 6 | $C_2H_5$ | Cl | 3,5-Dichlorphenyl | 157–158 |

Fortsetzung

| Nr. | $R^1$ | $R^2$ | $R^3$ | Fp. (°C) |
|---|---|---|---|---|
| A 7 | $C_2H_5$ | Cl | 4-Acetoxiphenyl | 164–165 |
| A 8 | $C_2H_5$ | Cl | 3-Acetarylphenyl | 89– 90 |
| A 9 | $C_2H_5$ | Cl | 3-Trifluormethylphenyl | 137–138 |
| A 11 | i-Propyl | Cl | Phenyl | 115–117 |
| A 12 | tert.-Butyl | Cl | Phenyl | 78– 79 |
| A 13 | Phenyl | Cl | Phenyl | 110–112 |
| A 14 | Phenyl | Cl | 3,5-Dichlorphenyl | 87– 88 |
| A 15 | Phenyl | Cl | 3-Trifluormethylphenyl | 96– 97 |
| A 16 | Phenyl | Chlor | Methyl | 105–106 |
| A 17 | 4-Chlorphenyl | Chlor | Phenyl | 193–195 |
| A 18 | Cyclohexyl | Chlor | 3-Trifluormethylphenyl | 112–114 |
| A 19 | Chlorhexyl | Brom | Phenyl | 113–115 |

Die folgenden Beispiele erläutern die Herstellung der neuen Pyridazinon-Derivate der Formel I. Gewichtsteile verhalten sich zu Volumenteilen wie kg zu Liter.

Beispiel 1

Zu einer Lösung von 16 Gew.-Teilen 1-Phenyl-4-(cyclohexylthio)-5-chlor-pyridazinon-6 in 100 Volumenteile Eisessig tropfte man 5,1 Volumenteile 30%iges (Gew.-%) Wasserstoffperoxid und rührte 12 Stunden bei 60°C nach. Dann rührte man das Reaktionsgemisch in 1000 Volumenteile Wasser ein, saugte den Niederschlag ab und wusch ihn mit Wasser. Nach Trocknen in Vakuum erhielt man 16,3 Gew.-Teile 1-Phenyl-4-(cyclohexylsulfinyl)-5-chlor-pyridazinon-6 vom Schmp. 128 bis 130°C (Verbindung Nr. 1).

$C_{16}H_{17}ClN_2O_2S$ (336,5):

| | | | | | |
|---|---|---|---|---|---|
| Ber.: | C 57,1 | H 5,1 | N 8,3 | Cl 10,5 | S 9,5 |
| Gef.: | C 57,2 | H 5,1 | N 8,2 | Cl 10,3 | S 9,6 |

Beispiel 2

Zu einer Lösung von 16 Gew.-Teilen 1-Phenyl-4-(cyclohexylthio)-5-chlor-pyridazinon-6 in 100 Volumenteilen Eisessig tropfte man bei Raumtemperatur 25 ml 30%iges Wasserstoffperoxid und rührte 5 Stunden bei 50°C nach. Nach dem Erkalten der Reaktionsmischung saugte man ab und wusch den Rückstand mit Wasser. Nach Trocknen in Vakuum erhielt man 13,8 Gew.-Teile 1-Phenyl-4-(cyclohexylsulfonyl)-5-chlor-pyridazinon-6 vom Schmp. 195 bis 196°C (Verbindung Nr. 2).

$C_{16}H_{17}ClN_2SO_3$ (352,5):

| | | | | | |
|---|---|---|---|---|---|
| Ber.: | C 54,5 | H 4,9 | N 7,9 | Cl 10,0 | S 9,1 |
| Gef.: | C 54,6 | H 4,8 | N 7,9 | Cl 9,9 | S 9,0 |

Entsprechend wurden diejenigen in der folgenden Tabelle angegebenen Verbindungen der Formel I hergestellt, deren Schmelzpunkt (Fp) angegeben sind. Ihre Struktur wurde durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden; es kann erwartet werden, daß sie aufgrund ihrer ähnlichen Konstitution ähnliche Wirkungen wie die näher untersuchten Verbindungen haben.

| Nr. | $R^1$ | $R^2$ | $R^3$ | X | Fp. (°C) |
|---|---|---|---|---|---|
| 3 | Ethyl | Chlor | Phenyl | SO | 142–143 |
| 4 | Ethyl | Chlor | Phenyl | $SO_2$ | 178–180 |
| 5 | Ethyl | Chlor | 4-Chlorphenyl | SO | 127–129 |
| 6 | Ethyl | Chlor | 4-Chlorphenyl | $SO_2$ | 181–183 |
| 7 | Ethyl | Chlor | 4-Acetoxiphenyl | SO | 149–151 |
| 8 | Ethyl | Chlor | 4-Acetoxiphenyl | $SO_2$ | 197–199 |
| 9 | Cyclohexyl | Chlor | 3-Trifluormethylphenyl | SO | 169–170 |
| 10 | Cyclohexyl | Chlor | 3-Trifluormethylphenyl | $SO_2$ | 158–160 |
| 11 | Ethyl | Chlor | 3-Methylphenyl | SO | Öl |
| 12 | Ethyl | Chlor | 4-Bromphenyl | $SO_2$ | 149–151 |
| 13 | Ethyl | Chlor | 4-Bromphenyl | SO | 157–158 |
| 14 | Ethyl | Chlor | 4-Bromphenyl | $SO_2$ | 165–166 |
| 15 | Phenyl | Chlor | 3,5-Dichlorphenyl | SO | 157–158 |
| 16 | Phenyl | Chlor | 3,5-Dichlorphenyl | $SO_2$ | 194–196 |
| 17 | Ethyl | Chlor | 3,5-Dichlorphenyl | SO | 99–101 |
| 18 | Ethyl | Chlor | 3,5-Dichlorphenyl | $SO_2$ | 142–143 |
| 19 | Phenyl | Chlor | 3-Trifluormethylphenyl | SO | 140–141 |
| 20 | Phenyl | Chlor | 3-Trifluormethylphenyl | $SO_2$ | 136–138 |
| 21 | Ethyl | Chlor | 3-Trifluormethylphenyl | SO | 120–121 |
| 22 | Ethyl | Chlor | 3-Trifluormethylphenyl | $SO_2$ | 139–140 |
| 23 | Phenyl | Chlor | Phenyl | SO | 122–123 |
| 24 | p-Chlorphenyl | Chlor | Phenyl | SO | 164–165 |
| 25 | tert.-Butyl | Chlor | Phenyl | $SO_2$ | 192–193 |
| 26 | i-Propyl | Chlor | Phenyl | $SO_2$ | 150–151 |
| 29 | Ethyl | Chlor | Isopropyl | SO | Öl |
| 30 | Ethyl | Chlor | Isopropyl | $SO_2$ | 135–136 |
| 31 | Cyclohexyl | Chlor | Isopropyl | SO | |
| 32 | Cyclohexyl | Chlor | Isopropyl | $SO_2$ | |
| 33 | Ethyl | Chlor | Methyl | SO | 83 |
| 34 | Ethyl | Chlor | Methyl | $SO_2$ | 138–139 |
| 35 | Ethyl | Chlor | Cyclohexyl | SO | |

Fortsetzung

| Nr. | $R^1$ | $R^2$ | $R^3$ | X | Fp. (°C) |
|---|---|---|---|---|---|
| 36 | Ethyl | Chlor | Cyclohexyl | $SO_2$ | |
| 37 | Cyclohexyl | Chlor | Cyclohexyl | SO | |
| 38 | Cyclohexyl | Chlor | Cyclohexyl | $SO_2$ | |
| 41 | Methyl | Chlor | 3,5-Dichlorphenyl | SO | |
| 42 | Methyl | Chlor | 3,5-Dichlorphenyl | $SO_2$ | |
| 43 | Cyclohexyl | Brom | Phenyl | SO | |
| 44 | Cyclohexyl | Brom | Phenyl | $SO_2$ | |

Die neuen Wirkstoffe zeigen eine starke Wirksamkeit gegen phytopathogene Pilze und sind insbesondere geeignet, Pflanzenkrankheiten zu verhüten oder zu heilen, wie zum Beispiel Botrytis cinerea an Reben, Erdbeeren und Paprika, Monilia fructigena an Äpfeln und Birnen, Plasmopara viticola an Reben, Pseudoperonospora an Hopfen, Peronospora helstedii an Sonnenblumen, Peronospora tabacina an Tabak, Septoria nodorum an Getreide, Septoria glycinea an Soja, Phytophthora infestans an Kartoffeln oder Tomaten, Alternaria solani an Tomaten und Kartoffeln, Cercospora beticola an Rüben, Pseudocercosporella herpotrichoides an Weizen und Gerste, Pyricularia oryzae an Reis, Pseudoperonospora cubensis an Gurken.

Die neuen Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollten in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha. Die neuen Verbindungen können auch im Materialschutz eingesetzt werden. Bei der Anwendung der Wirkstoffe im Materialschütz, z. B. als Fungizide für Anstrichfarben und Weich-Polyvinylchlorid, betragen die Aufwandmengen 0,05 bis 5% (Gew.-%) Wirkstoff, bezogen auf das Gesamtgewicht der zu konservierenden Farben bzw. des mikrozid auszurüstenden Polyvinylchlorids.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 5 mit 10 Gewichtsteilen N-Methyl-alpha-pyrrolidon und erhält eine Lösung, die zur Anwendung in Form kleinster Tropfen geeignet ist.

II. 10 Gewichtsteile der Verbindung des Beispiels 6 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 12 werden in einer Mischung gelöst, die aus 60 Gewichtsteilen Cyclohexanon, 30 Gewichtsteilen Isobutanol, 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 5 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 17 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 22 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung Nr. 18 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 25 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 26 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die neuen Fungizide können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Mangan-Zink-ethylendiamin-bis-dithiocarbamat und Zinkethylenbisdithiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;
heterocyclische Substanzen, wie
N-(1,1,2,2-Tetrachlorethylthio)-tetrahydrophthalimid,
N-Trichlormethylthio-tetrahydrophthalimid,
2-Heptadecyl-2-imidazolin-acetat,
2,4-Dichlor-6-(o-chloranilino)-s-triazin,
O,O-Diäthyl-phthalimidophonothioat,
5-Amino-1-(bis-(dimethylamino)-phosphinyl)-3-phenyl-1,2,4-triazol),
2,3-Dicyano-1,4-Dithioanthrachinon,
2-Thio-1,3-dithio-(4,5-b)-chinoxalin,
1-(Butylcarbamoyl)-2-benzimidazol-carbaminsäuremethylester,
4-(2-Chlorphenylhydrazono)-3-methyl-5-isoxazolon,
Pyridin-2-thio-1-oxid,

8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diylbis-(1-(2,2,2-trichlor-äthyl)-formamid,
2-(Thiazolyl-(4)-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol
und verschiedene Fungizide, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-[4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazol-yl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin,
1-(2,4-Dichlorphenyl)-4,4-dimethyl-2-(1,2,4-triazol-1-yl)-pentan-3-ol,
alpha-(2-Chlorphenyl)-alpha-(4-fluorphenyl)-5-pyrimidin-methanol,
alpha-(2-Chlorphenyl)-alpha-(4-chlorphenyl)-5-pyrimidin-methanol,
beta-([1,1'-Biphenyl]-4-yl-oxy)-alpha-(1,1-dimethylethyl)-1H-1,2,4-triazol-1-ethanol,
1-[2-(2,4-Dichlorphenyl)-4-ethyl-1,3-dioxolan-2-yl-methyl]-1H-1,2,4-triazol,
1-[2-(2,4-Dichlorphenyl)-4-propyl-1,3-dioxolan-2-yl-methyl]-1H-1,2,4-triazol,
1-[N-Propyl-N-[2-(2-(2,4,6-trichlorphenoxy)-ethyl]-carbamoyl]-imidazol,
2-Cyan-N-(ethylaminocarbonyl)-2-(methoxyimino)-acetamid
N-(1-Formylamido-2,2,2-trichlor-ethyl)-morpholin.

Für die folgenden Versuche wurden als bekannte Vergleichswirkstoffe die folgenden Verbindungen verwendet. A = N-Trichlormethylthiotetrahydrophthalimid (Chemical Week, June 21, 1972, Seite 46),

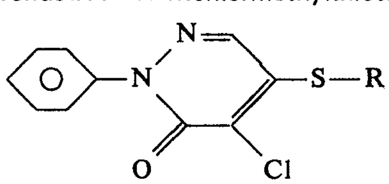
(DE-OS 1 445 475)

Vergleichswirkstoff B: R = 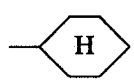

Vergleichswirkstoff C: $R = C_2H_5$

Vergleichswirkstoff D: $R = CH_3$

Vergleichswirkstoff E: 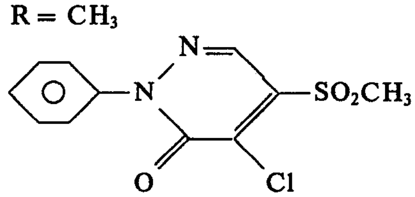

## Versuch 1

### Wirksamkeit gegen Botrytis cinerea an Paprika

Paprikasämlinge der Sorte »Neusiedler Ideal Elite« werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuchs zeigte, daß beispielsweise die Wirkstoffe 4, 5, 6, 12, 13, 14, 17, 18, 22, 25 und 26 bei Anwendung als 0,05%ige Spritzbrühe eine bessere fungizide Wirkung (beispielsweise 97%) hatten als die Wirkstoffe A, B, C, D und E (beispielsweise 70%). Bei Anwendung als 0,025 oder 0,0125%ige Spritzbrühe zeigten die Wirkstoffe 4, 12, 13 und 14 eine bessere fungizide Wirkung (beispielsweise 90%) als die Wirkstoffe D und E (beispielsweise 50%).

## Versuch 2

### Wirksamkeit gegen Phytophthora infestans an Tomaten

Blätter von Topfpflanzen der Sorte »Große Fleischtomate« werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis der Versuchs zeigte, daß die Wirkstoffe 2, 4, 5, 13, 14, 17, 18, 19, 22, 25 und 26 bei Anwendung als 0,025%ige Spritzbrühe eine bessere fungizide Wirkung (beispielsweise 97%) zeigten als die Wirkstoffe A, B, C und D (beispielsweise 50%). Bei Anwendung als 0,0125%ige Spritzbrühe zeigten die Wirkstoffe 2, 4, 13 und 14 eine bessere fungizide Wirkung (beispielsweise 97%) als die Wirkstoffe D und E (beispielsweise 70%).

Bei Anwendung als 0,05%ige Spritzbrühe zeigten die Wirkstoffe 2, 4, 13 und 14 eine sehr gute fungizide Wirkung (beispielsweise 100%).

## Versuch 3

### Wirksamkeit gegen Plasmopara viticola

Blätter von Topfreben der Sorte »Müller-Thurgau« werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuchs zeigte, daß die Wirkstoffe 2, 3, 4, 8, 24, 25 und 26 bei Anwendung als 0,025%ige Spritzbrühe eine gute fungizide Wirkung haben (beispielsweise 97%).

## Versuch 4

### Wirksamkeit gegen Septoria nodorum

Blätter von in Töpfen gewachsenen Weizenkeimlingen der Sorte »Jubilar« werden mit wäßriger Spritzbrühe, die 80% Wirkstoff und 20% Emulgiermittel in der Trockensubstanz enthält, besprüht, nach dem Antrocknen des Spritzbelages abgeschnitten und in Schalen mit wäßriger Benzimidazollösung (25 ppm) ausgelegt. Dann werden die Blätter mit einer wäßrigen Sporensuspension von Septoria nodorum infiziert und abgedeckt. Nach 7tägigem Stehen bei 20 bis 22°C wird das Ausmaß der Pilzentwicklung ermittelt.

Das Ergebnis des Versuchs zeigt, daß die Wirkstoffe 1, 2, 3, 23 und 24 bei Anwendung als 0,1%ige Spritzbrühe eine bessere fungizide Wirkung haben (beispielsweise 97%) als die Wirkstoffe B und C (beispielsweise 0%).

**Patentansprüche für die Vertragsstaaten: BE, CH, DE, FR, GB, IT, LI, NL, SE**

1. Pyridazinon-Derivate der Formel

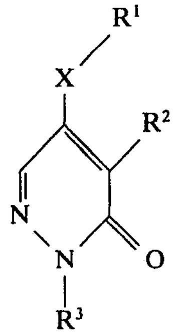

(I)

in der

$R^1$ Methyl, Ethyl, Propyl, i-Propyl, tert.-Butyl, Cyclohexyl, Phenyl oder p-Chlorphenyl,
$R^2$ Chlor oder Brom,
$R^3$ Wasserstoff, Methyl, Ethyl, i-Propyl, Cyclohexyl, Phenyl, 4-Chlorphenyl, 3,5-Dichlorphenyl, 4-Bromphenyl, 3-Trifluormethylphenyl, 3-Methylphenyl, 4-Methylphenyl, 4-Acetoxiphenyl oder 3-(Pentafluorethoxi)-phenyl und
X SO oder $SO_2$ bedeutet, mit der Maßgabe, daß $R^1$ nicht Methyl bedeutet, wenn X $SO_2$, $R^2$ Chlor und $R^3$ Phenyl bedeuten.

2. Fungizid, enthaltend ein Pyridazinon-Derivat gemäß Anspruch 1.
3. Fungizid, enthaltend einen inerten Trägerstoff und ein Pyridazinon-Derivat gemäß Anspruch 1.
4. Verfahren zur Herstellung eines Fungizids, dadurch gekennzeichnet, daß man einen inerten Trägerstoff vermischt mit einem Pyridazinon-Derivat gemäß Anspruch 1.
5. Verfahren zur Bekämpfung von Pilzen, dadurch gekennzeichnet, daß man die Pilze oder die durch Pilzbefall bedrohten Flächen, Pflanzen, Saatgüter oder Gegenstände behandelt mit einer fungizid wirksamen Menge eines Pyridazinon-Derivats gemäß Anspruch 1.
6. Verfahren zur Herstellung eines Pyridazinon-Derivats gemäß Anspruch 1, dadurch gekennzeichnet, daß man ein Pyridazinon-Derivat der Formel

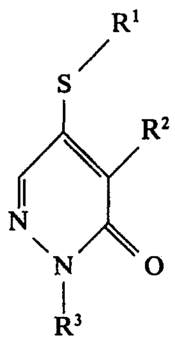

(II)

in der $R^1$, $R^2$ und $R^3$ die in Anspruch 1 genannten Bedeutungen haben, mit Wasserstoffperoxid oder organischen Persäuren, ggf. in Gegenwart eines Lösungs- oder Verdünnungsmittels, bei einer Temperatur im Bereich von 0 bis 100°C oxidiert.
7. Pyridazinon-Derivat gemäß Anspruch 1, dadurch gekennzeichnet, daß $R^1$ Ethyl oder Cyclohexyl, $R^2$ Chlor und $R^3$ Phenyl, 4-Chlorphenyl, 3,5-Dichlorphenyl oder 4-Bromphenyl bedeutet.
8. Fungizid gemäß Anspruch 2, dadurch gekennzeichnet, daß $R^1$ Ethyl oder Cyclohexyl, $R^2$ Chlor und $R^3$ Phenyl, 4-Chlorphenyl, 3,5-Dichlorphenyl oder 4-Bromphenyl bedeutet.

**Patentansprüche für den Vertragsstaat: AT**

1. Fungizid, enthaltend ein Pyridazinon-Derivat der Formel

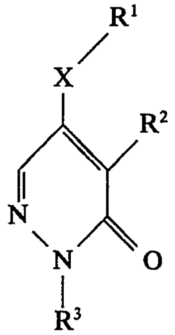

(I)

in der

$R^1$ Methyl, Ethyl, Propyl, i-Propyl, tert.-Butyl, Cyclohexyl, Phenyl oder p-Chlorphenyl,
$R^2$ Chlor oder Brom,
$R^3$ Wasserstoff, Methyl, Ethyl, i-Propyl, Cyclohexyl, Phenyl, 4-Chlorphenyl, 3,5-Dichlorphenyl, 4-Bromphenyl, 3-Trifluormethylphenyl, 3-Methylphenyl, 4-Methylphenyl, 4-Acetoxiphenyl oder 3-(Pentafluorethoxi)-phenyl und
X SO oder $SO_2$ bedeutet, mit der Maßgabe, daß $R^1$ nicht Methyl bedeutet, wenn X $SO_2$, $R^2$ Chlor und $R^3$ Phenyl bedeuten.

2. Fungizid, enthaltend einen inerten Trägerstoff und ein Pyridazinon-Derivat gemäß Anspruch 1.
3. Verfahren zur Herstellung eines Fungizids, dadurch gekennzeichnet, daß man einen inerten Trägerstoff vermischt mit einem Pyridazinon-Derivat gemäß Anspruch 1.
4. Verfahren zur Bekämpfung von Pilzen, dadurch gekennzeichnet, daß man die Pilze oder die durch Pilzbefall bedrohten Flächen, Pflanzen, Saatgüter oder Gegenstände behandelt mit einer fungizid wirksamen Menge eines Pyridazinon-Derivats gemäß Anspruch 1.
5. Verfahren zur Herstellung eines Pyridazinon-Derivats gemäß Anspruch 1, dadurch gekennzeichnet, daß man ein Pyridazinon-Derivat der Formel

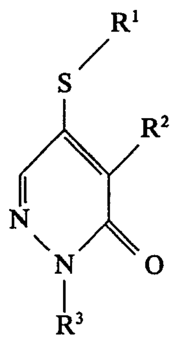

(II)

in der $R^1$, $R^2$ und $R^3$ die in Anspruch 1 genannten Bedeutungen haben, mit Wasserstoffperoxid oder organischen Persäuren, ggf. in Gegenwart eines Lösungs- oder Verdünnungsmittels, bei einer Temperatur im Bereich von 0 bis 100°C oxidiert.
6. Fungizid gemäß Anspruch 1, dadurch gekennzeichnet, daß $R^1$ Ethyl oder Cyclohexyl, $R^2$ Chlor und $R^3$ Phenyl, 4-Chlorphenyl, 3,5-Dichlorphenyl oder 4-Bromphenyl bedeutet.

**Claims for the Contracting States: BE, CH, DE, FR, GB, IT, LI, NL, SE**

1. A pyridazinone derivative of the formula

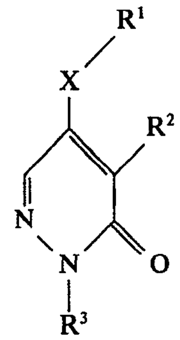

(I)

where

$R^1$ is methyl, ethyl, propyl, i-propyl, tert-butyl, cyclohexyl, phenyl or p-chlorophenyl,
$R^2$ is chlorine or bromine,
$R^3$ is hydrogen, methyl, ethyl, i-propyl, cyclohexyl, phenyl, 4-chlorophenyl, 3,5-dichlorophenyl, 4-bromophenyl, 3-trifluoromethylphenyl, 3-methylphenyl, 4-methylphenyl, 4-acetoxyphenyl or 3-(pentafluoroethoxy)-phenyl, and
X is SO or $SO_2$, with the proviso that $R^1$ is not methyl when X is $SO_2$, $R^2$ is chlorine and $R^3$ is phenyl.

2. A fungicide containing a pyridazinone derivative as claimed in claim 1.
3. A fungicide containing an inert carrier and a pyridazinone derivative as claimed in claim 1.
4. A process for producing a fungicide, wherein an inert carrier is mixed with a pyridazinone derivative as claimed in claim 1.
5. A process for combating fungi, wherein the fungi or the areas, plants, seed or objects threatened by fungal attack are treated with a fungicidally effective amount of a pyridazinone derivative as claimed in claim 1.
6. A process for preparing a pyridazinone derivative as claimed in claim 1, wherein a pyridazinone derivative of the formula

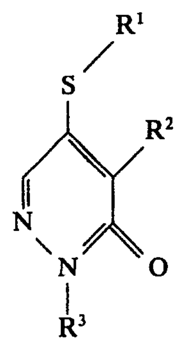

(II)

where $R^1$, $R^2$ and $R^3$ have the meanings given in claim 1, is oxidized with hydrogen peroxide or an organic per-acid, in the presence or absence of a solvent or diluent, at from 0 to 100°C.
7. A pyridazinone derivative as claimed in claim 1, wherein $R^1$ is ethyl or cyclohexyl, $R^2$ is chlorine, and $R^3$ is phenyl, 4-chlorophenyl, 3,5-dichlorophenyl or 4-bromophenyl.
8. A fungicide as claimed in claim 2, wherein $R^1$ is ethyl or cyclohexyl, $R^2$ is chlorine, and $R^3$ is phenyl, 4-chlorophenyl, 3,5-dichlorophenyl or 4-bromophenyl.

**Claims for the Contracting State: AT**

1. A fungicide containing a pyridazinone derivative of the formula

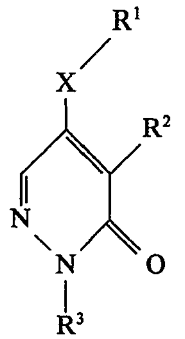

(I)

where

$R^1$ is methyl, ethyl, propyl, i-propyl, tert-butyl, cyclohexyl, phenyl or p-chlorophenyl,
$R^2$ is chlorine or bromine,
$R^3$ is hydrogen, methyl, ethyl, i-propyl, cyclohexyl, phenyl, 4-chlorophenyl, 3,5-dichlorophenyl, 4-bromophenyl, 3-trifluoromethylphenyl, 3-methylphenyl, 4-methylphenyl, 4-acetoxyphenyl or 3-(pentafluoroethoxy)-phenyl, and
X is SO or $SO_2$, with the proviso that $R^1$ is not methyl when X is $SO_2$, $R^2$ is chlorine and $R^3$ is phenyl.

2. A fungicide containing an inert carrier and a pyridazinone derivative as defined in claim 1.
3. A process for producing a fungicide, wherein an inert carrier is mixed with a pyridazinone derivative as defined in claim 1.
4. A process for combating fungi, wherein the fungi or the areas, plants, seed or objects threatened by fungal attack are treated with a fungicidally effective amount of a pyridazinone derivative as defined in claim 1.
5. A process for preparing a pyridazinone derivative as defined in claim 1, wherein a pyridazinone derivative of the formula

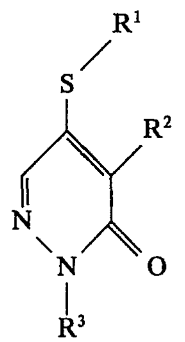

(II)

where $R^1$, $R^2$ and $R^3$ have the meanings given in claim 1, is oxidized with hydrogen peroxide or an organic per-acid, in the presence or absence of a solvent or diluent, at from 0 to 100°C.
6. A fungicide as claimed in claim 1, wherein $R^1$ is ethyl or cyclohexyl, $R^2$ is chlorine, and $R^3$ is phenyl, 4-chlorophenyl, 3,5-dichlorophenyl or 4-bromophenyl.

**Revendications pour les Etats contractants: BE, CH, DE, FR, GB, IT, LI, NL, SE**

1. Dérivés de pyridazinone de formule

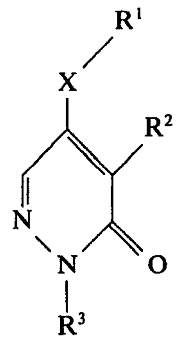

(I)

dans laquelle

$R^1$ représente méthyle, éthyle, propyle, i-propyle, tert-butyle, cyclohexyle, phényle ou p-chlorophényle,
$R^2$ chlore ou brome,
$R^3$ hydrogène, méthyle, éthyle, i-propyle, cyclohexyle, phényle, 4-chlorophényle, 3,5-dichlorophényle, 4-bromophényle, 3-trifluorométhylphényle, 3-méthylphényle, 4-méthylphényle, 4-acétoxyphényle ou 3-(pentafluoroéthoxy)-phényle, et
X représente SO ou $SO_2$, sous réserve que $R^1$ ne représente pas méthyle, si X représente $SO_2$, $R^2$, chlore et $R^3$, phényle.

2. Fongicide, contenant un dérivé de pyridazinone selon la revendication 1.
3. Fongicide, contenant un support inerte et un dérivé de pyridazinone selon la revendication 1.
4. Procédé de préparation d'un fongicide, caractérisé par le fait que l'on mélange un support inerte avec un dérivé de pyridazinone selon la revendication 1.
5. Procédé de lutte contre les champignons, caractérisé par le fait que l'on traite les champignons ou les surfaces, plantes, semences ou objets menacés par l'attaque de champignons, avec une quantité active du point de vue fongicide d'un dérivé de pyridazinone selon la revendication 1.
6. Procédé de préparation d'un dérivé de pyridazinone selon la revendication 1, caractérisé par le fait que l'on oxyde un dérivé de pyridazinone de formule

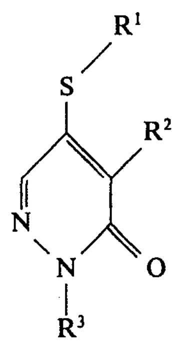

(II)

dans laquelle $R^1$, $R^2$ et $R^3$ ont les significations indiquées dans la revendication 1, avec de l'eau oxygénée ou un peracide organique, éventuellement en présence d'un solvant ou diluant, à une température comprise entre 0 et 100°C.
7. Dérivé de pyridazinone selon la revendication 1, caractérisé par le fait que $R^1$ représente éthyle ou cyclohexyle, $R^2$ chlore et $R^3$ phényle, 4-chlorophényle, 3,5-dichlorophényle ou 4-bromophényle.
8. Fongicide selon la revendication 2, caractérisé par le fait que $R^1$ représente éthyle ou cyclohexyle, $R^2$ chlore et $R^3$ phényle, 4-chlorophényle, 3,5-dichlorophényle ou 4-bromophényle.

**Revendications pour l'Etat contractant: AT**

1. Fongicide, contenant un dérivé de pyridazinone de formule

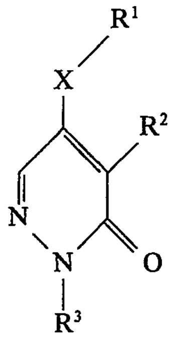

(I)

dans laquelle

$R^1$ représente méthyle, éthyle, propyle, i-propyle, tert-butyle, cyclohexyle, phényle ou p-chlorophényle,
$R^2$ chlore ou brome,
$R^3$ hydrogène, méthyle, éthyle, i-propyle, cyclohexyle, phényle, 4-chlorophényle, 3,5-dichlorophényle, 4-bromophényle, 3-trifluorométhylphényle, 3-méthylphényle, 4-méthylphényle, 4-acétoxyphényle ou 3-(pentafluoroéthoxy)-phényle, et
X représente SO ou $SO_2$, sous réserve que $R^1$ ne représente pas méthyle, si X représente $SO_2$, $R^2$, chlore et $R^3$, phényle.

2. Fongicide, contenant un support inerte et un dérivé de pyridazinone selon la revendication 1.
3. Procédé de préparation d'un fongicide, caractérisé par le fait que l'on mélange un support inerte avec un dérivé de pyridazinone selon la revendication 1.
4. Procédé de lutte contre les champignons, caractérisé par le fait que l'on traite les champignons ou les surfaces, plantes, semences ou objets menacés par l'attaque de champignons, avec une quantité active du point de vue fongicide d'un dérivé de pyridazinone selon la revendication 1.
5. Procédé de préparation d'un dérivé de pyridazinone selon la revendication 1, caractérisé par le fait que l'on oxyde un dérivé de pyridazinone de formule

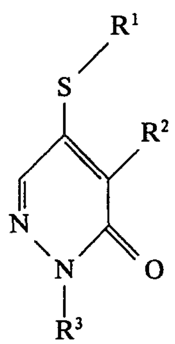

(II)

dans laquelle $R^1$, $R^2$ et $R^3$ ont les significations indiquées dans la revendication 1, avec de l'eau oxygénée ou un peracide organique, éventuellement en présence d'un solvant ou diluant, à une température comprise entre 0 et 100°C.
6. Fongicide selon la revendication 1, caractérisé par le fait que $R^1$ représente éthyle ou cyclohexyle, $R^2$ chlore et $R^3$ phényle, 4-chlorophényle, 3,5-dichlorophényle ou 4-bromophényle.